# EUROPEAN PATENT APPLICATION

(11) **EP 4 692 044 A1**
(43) Date of publication of application: **11.02.2026**
(21) Application number: 24780632.6
(22) Date of filing: 28.03.2024
(51) Int. Cl.: C07C 209/48, C07B 61/00, C07C 211/27

(54) **METHOD FOR PRODUCING XYLYLENEDIAMINE AND METHOD FOR CLEANING MAGNET STRAINER**

(30) Priority: 31.03.2023 JP 2023058898
(71) Applicant: MITSUBISHI GAS CHEMICAL COMPANY, INC., Chiyoda-ku Tokyo 100-8324 (JP)
(72) Inventor: SUGITA, Takuya, Niigata-shi, Niigata 950-3121 (JP); OHTANI, Takayuki, Niigata-shi, Niigata 950-3121 (JP); MUNEYASU, Kuniaki, Niigata-shi, Niigata 950-3121 (JP)
(74) Representative: Müller-Boré & Partner Patentanwälte PartG mbB
(86) International application number: PCT/JP2024/012623
(87) International publication number: WO 2024/204532

(57) **Abstract**

Provided is a xylylenediamine production method by hydrogenation of dicyanobenzene, the xylylenediamine production method comprising the steps of: hydrogenating the dicyanobenzene in the presence of a catalyst to obtain a mixed liquid containing at least a reaction product and at least a portion of the catalyst; and passing the mixed liquid through a magnet strainer to separate the catalyst by magnetic adsorption, wherein the magnet strainer comprises: a main body having an opening; a lid material placed on the opening of the main body; and a magnetic adsorption means disposed inside the main body and separated from the lid material.

## Description

### Technical Field

The present invention relates to a novel xylylenediamine production method and magnet strainer cleaning method.

### Background Art

Xylylenediamine is a compound that is useful as a starting material for polyamide resins, curing agents, and the like, and as an intermediate starting material for isocyanate resins and the like. For example, various methods using the hydrogenation of dicyanobenzene have been studied as xylylenediamine production methods. One example of the method for synthesizing xylylenediamine by hydrogenation of dicyanobenzene includes a method of performing hydrogenation reaction at two stages using dicyanobenzene as a starting material. This method can efficiently produce highly pure xylylenediamine. For example, a method for stably producing highly pure xylylenediamine with a low cyanobenzylamine content over a long period by incorporating a solid-liquid separation step into the method has been proposed as a process of producing xylylenediamine by performing the hydrogenation reaction at two stages (see, for example, the following Patent Literature 1).

### Citation List

### Patent Literature

Patent Literature 1: Japanese Patent No. 6806290

### Summary of Invention

### Technical Problem

As mentioned above, a purpose of the solid-liquid separation step performed as mentioned above includes the solid-liquid separation of a catalyst-derived catalyst powder resulting from a fixed-bed reactor at the time of xylylenediamine production. Such a catalyst is present in a reaction solution obtained by hydrogenation, and, for example, a magnet strainer using a magnet is adopted as a solid-liquid separation unit. In the case of using a magnet strainer, it is usually necessary to regularly clean and remove a fine catalyst powder attached to a rod-like magnet in order to prevent the catalyst from causing blockage at subsequent stages, after removing the catalyst powder for a predetermined time.

However, in a general magnet strainer, a rod-like magnet is placed in a strainer lid portion. Therefore, at the time of the cleaning of the magnet, it is necessary to lift the magnet portion together with a heavy lid using a chain block after opening a strainer main body. Furthermore, it is necessary to rapidly perform cleaning in order to safely remove the fine powder of the catalyst collected to the magnet strainer. Thus, there is a demand for the development of a convenient and safe strainer cleaning method.

To solve the problems mentioned above, an object of the present invention is to provide a xylylenediamine production method and a magnet strainer cleaning method that enable a magnet strainer to be cleaned rapidly and conveniently.

### Solution to Problem

The present inventor has completed the present invention by finding that use of a magnet strainer having a structure where a lid material and a magnetic adsorption means are separated from each other enables the magnetic adsorption means to be cleaned rapidly, conveniently, and safely by removing only the magnetic adsorption means at the time of cleaning.

<1> A xylylenediamine production method by hydrogenation of dicyanobenzene, the xylylenediamine production method comprising the steps of:
   hydrogenating the dicyanobenzene in the presence of a catalyst to obtain a mixed liquid containing at least a reaction product and at least a portion of the catalyst; and
   passing the mixed liquid through a magnet strainer to separate the catalyst by magnetic adsorption, wherein
   the magnet strainer comprises: a main body having an opening; a lid material placed on the opening of the main body; and a magnetic adsorption means disposed inside the main body and separated from the lid material.
<2> The xylylenediamine production method according to <1>, wherein the magnet strainer comprises, inside the main body, a guide that supports the magnetic adsorption means, and a bucket for removing solid matter in the mixed liquid.
<3> The xylylenediamine production method according to <1>, wherein the magnetic adsorption means comprises a magnet and a holding member that holds the magnet.
<4> The xylylenediamine production method according to <1>, wherein
   the magnet strainer comprises, inside the main body, a guide that supports the magnetic adsorption means, and a bucket for removing solid matter in the mixed liquid,
   the magnetic adsorption means comprises a magnet and a holding member that holds the magnet, and
   the holding member of the magnetic adsorption means is supported by the guide.
<5> The xylylenediamine production method according to any of <1> to <4>, further comprising the step of taking out the magnetic adsorption means separated from the lid material of the main body of the magnet strainer, followed by the cleaning of the magnetic adsorption means.
<6> A magnet strainer cleaning method for removing a catalyst by magnetic adsorption from a mixed liquid containing a reaction product and the catalyst, the mixed liquid being obtained by hydrogenation of dicyanobenzene in the presence of the catalyst, wherein
   the magnet strainer comprises: a main body having an opening; a lid material placed on the opening of the main body; and a magnetic adsorption means disposed inside the main body and separated from the lid material, and
   the magnetic adsorption means separated from the lid material is taken out of the main body of the magnet strainer, followed by the cleaning of the magnetic adsorption means.

### Advantageous Effect of Invention

The present invention can provide a xylylenediamine production method and a magnet strainer cleaning method that enable a magnet strainer to be cleaned rapidly and conveniently.

### Brief Description of Drawings

[Figure 1] Figure 1 is a schematic view showing the configuration of the magnet strainer according to the present embodiment.
[Figure 2] Figure 2 is a schematic view showing a state in which a lid material is closed in the magnet strainer.
[Figure 3] Figure 3 is a schematic view for illustrating the cleaning step according to the present embodiment.
[Figure 4] Figure 4 is a flow chart showing the basic flow of the present embodiment.

### Description of Embodiments

The xylylenediamine production method of the present embodiment (hereinafter, also referred to as the "production method of the present embodiment") is a production method for producing xylylenediamine by hydrogenation of dicyanobenzene, comprising the steps of: hydrogenating the dicyanobenzene in the presence of a catalyst to obtain a mixed liquid containing at least a reaction product and at least a portion of the catalyst (hereinafter, also referred to as the "first hydrogenation step"); and passing the mixed liquid through a magnet strainer to separate the catalyst by magnetic adsorption (hereinafter, also referred to as the "solid-liquid separation step"), wherein the magnet strainer comprises: a main body having an opening; a lid material placed on the opening of the main body; and a magnetic adsorption means disposed inside the main body and separated from the lid material. The production method of the present embodiment comprises at least the first hydrogenation step and the solid-liquid separation step and may further comprise, if necessary, a cleaning step, a liquid ammonium removal step, a second hydrogenation step, and the like.

The magnet strainer cleaning method of the present embodiment (hereinafter, also referred to as the "the cleaning method of the present embodiment") is a magnet strainer cleaning method for removing a catalyst by magnetic adsorption from a mixed liquid containing a reaction product and the catalyst, the mixed liquid being obtained by hydrogenation of dicyanobenzene in the presence of the catalyst, wherein the magnet strainer comprises: a main body having an opening; a lid material placed on the opening of the main body; and a magnetic adsorption means disposed inside the main body and separated from the lid material, and the magnetic adsorption means separated from the lid material is taken out of the main body of the magnet strainer, followed by the cleaning of the magnetic adsorption means. The cleaning method of the present embodiment corresponds to the cleaning step of the production method of the present embodiment mentioned later.

According to the production method and the cleaning method of the present embodiment, a mixed liquid containing a reaction product and at least a portion of a catalyst, obtained by hydrogenation of dicyanobenzene in the presence of the catalyst is applied to a magnet strainer comprising a lid material and a magnetic adsorption means separated from the lid material so that the catalyst in the liquid can be removed by magnetic adsorption. On the other hand, as mentioned above, the magnet strainer, after being used in the removal of a catalyst powder for a predetermined time, is regularly subjected to a cleaning step for removing the catalyst attached to a magnet of the magnetic adsorption means in order to prevent the catalyst from causing blockage at subsequent stages. In the magnet strainer according to the present embodiment, the magnetic adsorption means is another member separated from the lid material, which is different from a conventional configuration. Therefore, the weight of the lid material itself can be decreased, and the lid material can be easily opened and closed in the cleaning of the magnet strainer. Furthermore, the magnet strainer, for its cleaning, eliminates the need of raising the magnetic adsorption means together with a heavy lid using a chain block. Thus, only the magnetic adsorption means can be taken out of the magnet strainer. This can drastically reduce work burdens at the time of magnet strainer cleaning. Moreover, the magnet strainer eliminates the need of making preparations for operation using a chain block and can thus be cleaned rapidly and conveniently while the magnet strainer can also reduce physical burdens on persons who clean the magnet strainer. In the cleaning of the magnet strainer, safety treatment such as water spraying to a catalyst powder attached to magnet surface of the magnetic adsorption means is carried out in order to suppress the heat generation of the catalyst. The production method and the cleaning method of the present embodiment also permit rapid operation of spraying water to a catalyst powder because the magnetic adsorption means can be conveniently taken out. Thus, cleaning operation can be more safely performed.

Thus, the production method and the cleaning method of the present embodiment enable a magnet strainer to be cleaned rapidly and conveniently and therefore enable xylylenediamine to be efficiently produced.

The production method of the present embodiment adopts two stages of hydrogenation reaction and involves the solid-liquid separation step between the first hydrogenation step and the second hydrogenation step, and can thereby continue to stably produce highly pure xylylenediamine for a long time without changing conditions of the second hydrogenation reaction. Although the reason for this is not certain, it is believed that a fine powder of a catalyst coming out of a reactor used in the first hydrogenation reaction is removed in the solid-liquid separation step before being supplied to a reactor for use in the second hydrogenation reaction, whereby the fine catalyst powder can be prevented from being accumulated in the reactor for use in the second hydrogenation reaction, and furthermore, the degeneration of xylylenediamine caused by the accumulated fine catalyst powder and the aggregation of the catalyst promoted thereby can be effectively suppressed. As a result, the production method of the present embodiment can presumably continue to stably produce highly pure xylylenediamine for a long time without changing conditions of the second hydrogenation reaction

### <<Magnet strainer>>

First, the magnet strainer for use in the production method and the cleaning method of the present embodiment will be described. The magnet strainer according to the present embodiment comprises: a main body having an opening; a lid material placed on the opening of the main body; and a magnetic adsorption means disposed inside the main body and separated from the lid material. In this context, the "magnetic adsorption means separated from the lid material" means that the lid material and the magnetic adsorption means are not connected, for example, to each other directly or via another member and are each independently and separately mountable to the magnet strainer.

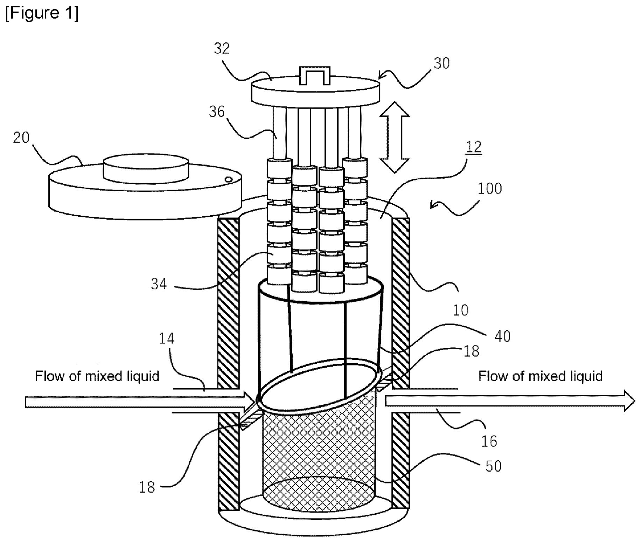

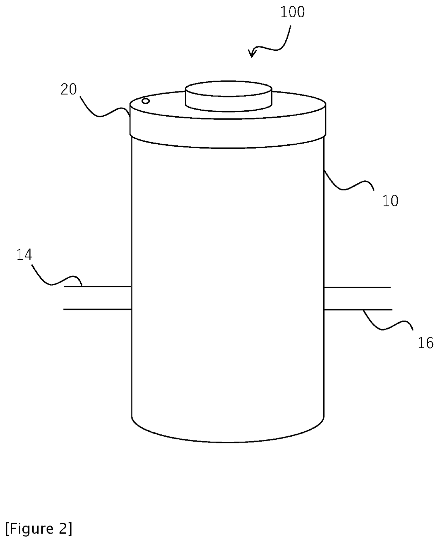

The magnet strainer according to the present embodiment will be described with reference to Figures 1 and 2. Figure 1 is a schematic view showing one example of the configuration of the magnet strainer according to the present embodiment. Figure 2 is a schematic view showing a state in which a lid material is closed in a magnet strainer 100. As show in Figure 1, the magnet strainer 100 comprises: a main body 10 having an opening 12; a lid material 20 placed on the opening 12 of the main body 10; and a magnetic adsorption means 30 disposed inside the main body 10 and separated from the lid material 20, and further comprises, inside the main body 10, a guide 40 that supports the magnetic adsorption means 30, and a bucket 50 for removing solid matter in the mixed liquid. In the magnet strainer 100, the magnetic adsorption means 30 comprises a magnet 34 and a holding member 32 that holds the magnet 34, and the holding member 32 of the magnetic adsorption means 30 is supported by the guide 40. In this context, Figure 1 shows a state in which the lid material 20 is moved so that the opening 12 of the main body 10 is opened, and is a cross-sectional view in which the wall of the main body 10 is partially omitted in order to show the inside. Each of Figures 1 and 2 is a schematic view, and the configuration of the magnet strainer according to the present embodiment is not limited to that shown in Figure 1. Likewise, the size or shape of each member or the ratio between the members is not limited thereto.

The magnet strainer 100 is an apparatus that is placed in the solid-liquid separation step according to the present embodiment, and separates at least the catalyst by magnetic adsorption from the mixed liquid obtained in the first hydrogenation step, by passing the mixed liquid through the main body 10. In the form shown in Figure 1, the magnetic adsorption means 30, the guide 40, and the bucket 50 are each placed so as to be capable of being taken out of the inside of the main body 10 via the opening 12 in the cleaning step.

The main body 10 has the opening 12 and has internal space where the magnetic adsorption means 30 and the like can be placed. As shown in Figure 2, in the solid-liquid separation step, the lid material 20 is in a closed state and covers the opening 12 of the main body 10. As shown in Figures 1 and 2, the main body 10 has an inlet 14 and an outlet 16. In the solid-liquid separation step, the mixed liquid obtained in the first hydrogenation step is supplied to the inside of the main body 10 from the inlet 14. Subsequently, the mixed liquid supplied to the inside of the main body 10 comes into contact with the magnetic adsorption means 30 and passes through a filter disposed in the bucket 50, thereby removing solid matter in the mixed liquid. The mixed liquid that has come into contact with the magnetic adsorption means 30 and passed through the bucket 50 is discharged to the outside of the main body 10 from the outlet 16.

The opening 12 is disposed on the upper side in the direction of gravitational force (upper side of the paper surface in Figure 1) in the main body 10. Its inside diameter is set such that the opening is opened in the cleaning step and enables the magnetic adsorption means 30 placed in the inside to be taken out of the opening 12.

The lid material 20 is mounted pivotally in the horizontal direction to the edge of the opening 12 on the upper face of the main body 10. In the cleaning step, the opening 12 is opened and closed by the pivotal movement of the lid material 20. However, the opening and closing mechanism of the lid material 20 is not limited to the pivotal movement in the horizontal direction, and other opening and closing mechanisms may be used, for example, sliding in the horizontal direction or opening in the vertical direction through a hinge, as long as the opening 12 can be easily opened and closed. The lid material 20 may be fixed to the main body 10 through a bolt or the like in a state in which the opening 12 is covered with the lid material 20 in the solid-liquid separation step.

The magnetic adsorption means 30, as shown in Figure 1, comprises the magnet 34 and the holding member 32 that holds the magnet 34, and the magnet 34 can come into contact with the mixed liquid supplied to the main body 10 and thereby remove the catalyst in the mixed liquid by adsorption. As shown in Figure 1, a plurality of magnet members 36 are mounted on the lower side in the direction of gravitational force (lower side of the paper surface in Figure 1) in the holding member 32, and a plurality of magnets 34 are disposed in each magnet member 36. The magnetic force of the magnetic adsorption means 30 is desirably selected as magnetic force capable of removing a fine catalyst powder in consideration of the size of the magnetic adsorption means 30, a flow rate of a solution, and the like, and specifically, is preferably 0.1 to 3 T, more preferably 0.5 to 2 T, per magnet member 36.

In Figure 1, the magnet members 36 has a rod-like shape. In this context, Figure 1 shows a state after the magnetic adsorption means 30 is taken out of the main body 10. In the solid-liquid separation step, the magnetic adsorption means 30 is housed inside the main body 10. Specifically, the holding member 32 of the magnetic adsorption means 30 is supported by the guide 40 such that at least a portion of sites having the magnets 34 in the magnet members 36 is accommodated in the bucket 50. The magnetic adsorption means 30 shown in Figure 1 is configured such that the magnets 34 are disposed via the rod-like magnet members 36 on the holding member 32. However, the configuration of the magnetic adsorption means according to the present embodiment is not limited to this form. Other configurations may be used, for example, a form in which rod-like magnets are mounted directly or indirectly to the holding member.

As shown in Figure 1, the magnet strainer 100 can comprise, if necessary, inside the main body 10, the guide 40 that supports the magnetic adsorption means 30, and the bucket 50 for removing solid matter in the mixed liquid. The guide 40 plays a role in supporting the holding member 32 of the magnetic adsorption means 30 in the main body 10. This can disperse the weight of the magnetic adsorption means 30 to the outer periphery when the bucket 50 is placed, as compared with the case where the magnetic adsorption means 30 is supported directly by the bucket 50. Thus, burdens on the bucket 50 can be reduced. The guide 40 can be constituted by a plurality of ring-like members in combination and is not limited by its configuration as long as the guide can support the holding member 32 of the magnetic adsorption means 30 in the main body 10. In Figure 1, the shape of the guide 40 is a shape diagonally cut at its lower part according to the upper shape of the bucket 50. However, the shape of the guide 40 is not limited thereto.

The bucket 50 is a member having a filter and can remove, through the filter, solid matter in the mixed liquid passing through the bucket 50. In Figure 1, the bucket 50 has a diagonally cut shape of a cylindrical member when viewed cross-sectionally. Specifically, the shape of the bucket 50 is configured such that a wall height on the inlet 14 side is smaller than the inlet 14 and a wall height on the outlet 16 side is larger than the outlet 16 so as to be capable of collecting solid matter in the mixed liquid within the bucket 50. During the course in which the mixed liquid supplied from the inlet 14 flows toward the outlet 16, solid matter in the mixed liquid is thereby collected by the filter on the outlet 16 side of the bucket 50 so that the solid matter can be captured within the bucket 50. However, the bucket 50 is not limited by this shape. The shape is not particularly limited as long as the shape can remove solid matter in the mixed liquid through the filter.

As shown in Figure 1, the inner wall of the main body 10 is provided with a ring-like projection 18 along the inner wall. The bucket 50 is supported by the projection 18 such that the bucket is fixed in midair in the main body 10. This can also disperse the weight of the magnetic adsorption means 30 to the inner periphery of the main body 10 than the projection 18, as compared with the case where the magnetic adsorption means 30 is supported directly by the bucket 50. Thus, burdens on the bucket 50 can be reduced. The bucket 50 thus supported by the projection 18 so as to be fixed in midair enables the whole amount of the mixed liquid to pass through the bucket 50 because liquid flow also occurs from the undersurface of the bucket 50.

### <<Cleaning method (cleaning step)>>

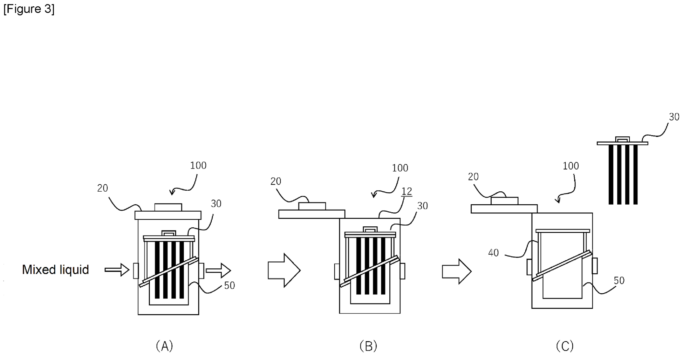

Next, the cleaning method (cleaning step) of the present embodiment will be described with reference to Figure 3. Figure 3 is a schematic view for illustrating the cleaning step according to the present embodiment. In Figure 3, Figure 3(A) shows the state of the magnet strainer 100 in the solid-liquid separation step. The cleaning step is the step of taking out the magnetic adsorption means separated from the lid material of the main body of the magnet strainer, followed by the cleaning of the magnetic adsorption means. For the production method of the present embodiment, it is preferred to regularly carry out the cleaning step for removing the catalyst attached to the magnet of the magnetic adsorption means in order to prevent the catalyst from causing blockage at subsequent stages, after removing the catalyst powder for a predetermined time by the solid-liquid separation step.

In the solid-liquid separation step, the mixed liquid obtained in the first hydrogenation step is supplied to the inside of the magnet strainer 100, as indicated by the arrow in Figure 3(A). In the magnet strainer 100, the mixed liquid comes into contact with the magnetic adsorption means 30 so that the catalyst in the mixed liquid is removed. Solid matter other than the catalyst in the mixed liquid is removed through the filter of the bucket 50 when passing through the bucket 50. The mixed liquid from which the solid matter such as the catalyst has been removed is discharged to the outside of the magnet strainer 100, as indicated by the arrow in Figure 3(A).

In Figure 3, Figures 3(B) and 3(C) each show the state of the magnet strainer 100 in the cleaning step. In the cleaning step, as shown in Figure 3(B), the opening 12 of the magnet strainer 100 is opened by the pivotal movement in the horizontal direction of the lid material 20 after cessation of supply of the mixed liquid. Subsequently, as shown in Figure 3(C), the magnetic adsorption means 30 is taken out of the magnet strainer 100, and the catalyst in a powder form and the like adsorbed to the magnet are removed. In this respect, it is preferred to spray water or the like to the catalyst adsorbed to the magnet immediately after taking out the magnetic adsorption means 30 of the magnet strainer 100, in order to suppress the heat generation of the catalyst by exposure to outside air, as mentioned above.

In the cleaning step, it is preferred to take out the guide 40 and the bucket 50, together with the magnetic adsorption means 30, of the magnet strainer 100 and remove solid matter collected by the bucket 50.

After the completion of cleaning, the magnetic adsorption means 30 and the like are brought back to a predetermined position in the magnet strainer 100, and the solid-liquid separation step is carried out again by the closing of the lid material 20.

In the cleaning method (cleaning step) of the present embodiment, only one magnet strainer may be used, or a plurality of magnet strainers may be used. In the case of using a plurality of magnet strainers, the magnet strainers may be arranged in tandem or in parallel with respect to the flow of the mixed liquid supplied from the first hydrogenation step. Alternatively, tandem and parallel arrangements may be combined. In the case of using a plurality of magnet strainers, valves placed at the inlet and the outlet of the magnet strainer to be cleaned can be closed to shift the flow of the mixed liquid so as to supply the mixed liquid from the first hydrogenation step only to the magnet strainer(s) excluded from the cleaning. A xylylenediamine production step can thereby be continuously performed without the need of cessation by the implementation of the cleaning step.

### <<Xylylenediamine production method>>

The production method of the present embodiment, as mentioned above, comprises the first hydrogenation step and the solid-liquid separation step and preferably further comprises the cleaning step of cleaning the inside of the magnet strainer (cleaning of the magnetic adsorption means) used in the solid-liquid separation step. In addition, an ammonia separation step or a purification step can be established, if necessary.

The production method of the present embodiment can adopt, for example, two stages of hydrogenation reaction. When the second hydrogenation step is established in addition to the first hydrogenation step, cyanobenzylamine produced as a by-product in the first hydrogenation step can be efficiently converted to xylylenediamine by hydrogenation in the second hydrogenation step. The production method of the present embodiment involving the solid-liquid separation step between the first hydrogenation step and the second hydrogenation step can continue to stably produce highly pure xylylenediamine for a long period without changing conditions of the second hydrogenation reaction.

One preferred example of the production method of the present embodiment includes a method comprising the following steps (1) to (5):
(1) a first hydrogenation step of hydrogenating a mixture of dicyanobenzene and a solvent containing liquid ammonia in the presence of a catalyst in a fixed-bed reactor to obtain a mixed liquid containing a reaction product and the catalyst;
(2) an ammonia separation step of separating and removing liquid ammonia contained in the mixed liquid;
(3) a solid-liquid separation step of passing the mixed liquid through the magnet strainer for solid-liquid separation to remove a solid component such as the catalyst;
(4) a second hydrogenation step of hydrogenating the mixed liquid from which the solid component has been removed in a fixed-bed reactor; and
(5) the step of taking out the magnetic adsorption means separated from the lid material of the main body of the magnet strainer, followed by the cleaning of the magnetic adsorption means.

In the production method of the present embodiment, the implementation of the solid-liquid separation step after the ammonia separation step is industrially advantageous because the amount of the liquid injected in the solid-liquid separation step is decreased; thus, a small facility and a low pressure can be attained. However, the ammonia separation step (2) and the solid-liquid separation step (3) mentioned above may be performed in a reversed order, and the ammonia separation step may be carried out after the solid-liquid separation step.

Hereinafter, each step of the production method of the present embodiment will be described in detail.

### <First hydrogenation step>

The first hydrogenation step is the step of hydrogenating dicyanobenzene in the presence of a catalyst to obtain a mixed liquid containing at least a reaction product and at least a portion of the catalyst. In this step, for example, a solution containing dicyanobenzene and a solvent containing liquid ammonia can be hydrogenated in a fixed-bed reactor to obtain a reaction product.

### (Dicyanobenzene)

The dicyanobenzene for use in this step is not particularly limited and may be obtained by any method. For example, dicyanobenzene obtained through the ammoxidation reaction of xylene is industrially preferred.

### -Ammoxidation reaction-

The ammoxidation reaction for obtaining dicyanobenzene may be carried out by a known method. Specifically, this reaction can be performed by reacting a reaction starting material having a mixture of a catalyst, xylene, oxygen, and ammonia. The ammoxidation reaction may be performed in a fluidized-bed or fixed-bed system. A known catalyst can be used as the ammoxidation catalyst. The ammoxidation catalyst preferably contains vanadium or chromium and more preferably contains vanadium and chromium.

The amount of ammonia is preferably 2 to 20 mol, more preferably 6 to 15 mol, based on 1 mol of xylene. When the amount of ammonia falls within the range mentioned above, the yield of dicyanobenzene is favorable and its space time yield is also high. Oxygen may be diluted with nitrogen, carbon dioxide, or the like and used as an oxygen-containing gas.

Air is preferably used as the oxygen-containing gas. The amount of oxygen is preferably 3 mol or more, more preferably 4 to 100 mol, based on 1 mol of xylene. When the amount of oxygen falls within the range mentioned above, the yield of dicyanobenzene is favorable and its space time yield is also high. The reaction temperature is preferably 300 to 500°C, more preferably 330 to 470°C. When the temperature falls within the range mentioned above, the conversion rate of xylene is favorable, by-products are suppressed, and dicyanobenzene can be produced with a favorable yield. The pressure is preferably ordinary pressure (atmospheric pressure) to 300 kPa, and the space velocity (gas hourly space velocity = GHSV) of the reaction starting material is preferably 500 to 5000 h⁻¹.

Dicyanobenzene obtained through the reaction can be collected and used as a starting material in the first hydrogenation step. As for the collection of dicyanobenzene, an ammoxidation reaction product in a gas state may be cooled to a temperature at which dicyanobenzene is deposited, followed by collection, or an ammoxidation reaction product in a gas state may be collected with water or an appropriate organic solvent. The organic solvent for use in collection is preferably one or more types of organic solvents that have high solubility of dicyanobenzene and are inert to dicyanobenzene. Among them, tolunitrile is further preferred. The dicyanobenzene collection solution may be used directly in the first hydrogenation step. Before use in the first hydrogenation step, a portion or the whole of a component having a lower boiling point (low boiling point component) than that of dicyanobenzene containing the organic solvent is preferably separated by distillation. Such separation of the low boiling point component by distillation can produce dicyanobenzene while the collected organic solvent can be used in the collection of a reaction product again. A portion or the whole of a component having a higher boiling point (high boiling point component) than that of dicyanobenzene may also be separated by distillation or extraction.

For example, any of three isomers phthalonitrile (1,2-dicyanobenzene), isophthalonitrile (1,3-dicyanobenzene), and terephthalonitrile (1,4-dicyanobenzene) can be used as dicyanobenzene. The ammoxidation method mentioned above can produce the desired dicyanobenzene by appropriately selecting the corresponding xylene, i.e., ortho-xylene, meta-xylene, or para-xylene. In the production method of the present embodiment, preferably, the dicyanobenzene in the first hydrogenation step is isophthalonitrile, and the resulting xylylenediamine is meta-xylylenediamine.

### (Solvent containing liquid ammonia)

In this step, dicyanobenzene can be dissolved in a solvent containing liquid ammonia and then hydrogenated in a liquid phase in the presence of a catalyst.

Examples of the solvent containing liquid ammonia include (i) liquid ammonia, (ii) a mixed solvent of liquid ammonia and aromatic hydrocarbon, (iii) a mixed solvent of liquid ammonia and xylylenediamine, and (iv) a mixed solvent of liquid ammonia, aromatic hydrocarbon, and xylylenediamine. One aromatic hydrocarbon or a mixture of two or more aromatic hydrocarbons may be used.

The liquid ammonia concentration in the solvent is preferably higher, specifically, more preferably 60% by mass or more, further preferably 100% by mass, from the viewpoint of enhancing the yield of hydrogenation reaction of dicyanobenzene. The amount of the solvent containing liquid ammonia in this step is not particularly limited and is preferably 1 to 99 parts by mass, more preferably 3 to 66 parts by mass, further preferably 5 to 49 parts by mass, based on 1 part by mass of dicyanobenzene. When the amount of the solvent falls within the range mentioned above, small energy is required for solvent collection and is economically advantageous and the selectivity of xylylenediamine in hydrogenation reaction is also favorable.

The dissolution unit of dicyanobenzene in the solvent containing liquid ammonia is not particularly limited, and a mixing machine such as a static mixer is used. In addition, dicyanobenzene is preferably mixed with and dissolved in the solvent containing liquid ammonia in a dissolution tank in advance, from the viewpoint of preventing the attachment of a deposited insoluble component to the mixing machine or the like. In this respect, dicyanobenzene in a melted state and the solvent containing liquid ammonia are preferably supplied into the dissolution tank and dissolved. If necessary, a stirring unit may be used.

When dicyanobenzene is dissolved in the solvent containing liquid ammonia, the internal pressure and temperature of the dissolution tank are preferably adjusted such that the solution maintains a liquid phase. For example, the internal pressure of the dissolution tank is preferably 0.5 to 15 MPa, more preferably 0.7 to 10 MPa, further preferably 1 to 8 MPa. The internal solution temperature of the dissolution tank is preferably 3 to 140°C, more preferably 5 to 120°C, further preferably 10 to 100°C.

### -Fixed-bed reactor-

The hydrogenation reaction in this step can be performed using a fixed-bed reactor. The fixed-bed reaction may be any of batch reaction and continuous reaction, and continuous reaction is preferred for sufficiently exerting the advantageous effects of the present invention.

In the case of performing continuous reaction, a circulation scheme of continuously putting a portion of a hydrogenation reaction liquid obtained from a hydrogenation reactor exit back into the hydrogenation reactor may be used, or the circulation scheme may be combined with a one-pass scheme, as described in Japanese Patent Laid-Open No. 2008-31155. In the case of performing continuous reaction, the space velocity (liquid hourly space velocity = LHSV) of the reaction starting material is preferably 0.1 to 10 h⁻¹.

In the case of performing batch reaction, the hydrogenation reaction time is preferably 0.5 to 8 hours.

### (Catalyst)

For example, a known support metal catalyst, non-supported metal catalyst, Raney catalyst, sponge catalyst, or noble metal catalyst can be used as the catalyst for use in this step. Particularly, a catalyst containing a ferromagnet nickel and/or cobalt is preferably used. The amount of the catalyst used can be an amount that is used in known liquid-phase hydrogenation of dicyanobenzene.

### (Reaction condition in first hydrogenation step)

Hydrogen for use in this step may contain impurities that are not involved in the reaction, for example, methane and nitrogen. However, a high concentration of the impurities in hydrogen requires elevating the total pressure of the reaction for securing a necessary hydrogen partial pressure, and is thus industrially disadvantageous. Therefore, the hydrogen concentration in a gas containing hydrogen is preferably 50% by mol or more, more preferably 80% by mol or more.

For efficiently producing xylylenediamine in this step, it is preferred to enhance the degree of progression of the hydrogenation reaction of a nitrile group into an aminomethyl group, and it is preferred to select reaction conditions under which the concentrations of dicyanobenzene and cyanobenzylamine are kept low in the mixed liquid containing a reaction product obtained after the hydrogenation reaction. Specifically, the amount of cyanobenzylamine based on xylylenediamine in the reaction product obtained after the hydrogenation reaction is preferably kept at 5.0% by mass or less, more preferably at 1.0% by mass or less, further preferably at 0.2% by mass or less. The conversion rate of dicyanobenzene is preferably 99.50% or more, more preferably 99.90% or more, further preferably 99.95% or more.

The pressure and temperature of the hydrogenation reaction are preferably adjusted such that the solution maintains a liquid phase. The temperature of the hydrogenation reaction is preferably 20 to 200°C, more preferably 30 to 150°C, further preferably 40 to 120°C. The hydrogen pressure is preferably 1 to 30 MPa, more preferably 2 to 25 MPa, further preferably 3 to 20 MPa.

### <Ammonia separation step>

The ammonia separation step is the step of separating and removing liquid ammonia contained in the mixed liquid. The ammonia separation step may be performed between the first hydrogenation step and the solid-liquid separation step or may be performed between the solid-liquid separation step and the second hydrogenation step.

In this step, the whole or a portion of liquid ammonia in the solvent containing liquid ammonia used in the preceding step is separated and removed. When the solvent containing liquid ammonia contains a solvent other than liquid ammonia, such as aromatic hydrocarbon, only liquid ammonia may be separated and removed, or liquid ammonia and the solvent other than liquid ammonia may be separated and removed at the same time. Liquid ammonia and the solvent other than liquid ammonia are preferably separated and removed at the same time from an industrial standpoint.

In this step, the method for separating and removing liquid ammonia is not particularly limited and is preferably, for example, a method of separating and removing liquid ammonia by distillation. The distillation is preferably performed under a pressurization condition, and in this respect, the pressure is preferably 0.2 to 3 MPa. The temperature in the distillation is preferably 50 to 200°C, more preferably 70 to 180°C. A known distillation apparatus such as a packed column, a plate column, or a flash drum may be used as a distillation apparatus that can be used in this step. The distillation can be carried out in a bath or continuous manner.

The amount of ammonia in the mixed liquid obtained after the distillation of this step is preferably 1.0% by mass or less. The amount of ammonia of 1.0% by mass or less is economically advantageous because elevation in partial pressure of the solution after the distillation can be prevented and a high pressure reactor is unnecessary for subsequent processes.

### <Solid-liquid separation step>

The solid-liquid separation step is the step of passing the mixed liquid through the magnet strainer to separate the catalyst by magnetic adsorption, and is the step of removing solid matter such as the catalyst in the mixed liquid.

The solid component in this step is mainly an insoluble component resulting from the first hydrogenation step, and the main component is a catalyst powder derived from the catalyst used in the first hydrogenation step. Specifically, a fine catalyst powder having a particle size of 1 to 500 µm is considered as the main component.

In this step, the solid-liquid separation is performed by magnetic adsorption using the magnet strainer according to the present embodiment, as mentioned above. The production method of the present embodiment adopts magnetic adsorption and is thereby capable of efficiently removing a fine catalyst powder derived from the catalyst that employs a ferromagnetic component such as nickel or cobalt. Thus, a solution can be obtained in which the fine catalyst powder rarely remains. In this step, the magnetic adsorption using the magnet strainer may be used in combination with a known adsorption (adsorption through intermolecular force, etc.), filtration, or precipitation unit, such as filtration using a filter.

The filtering equipment for use in the filtration is not particularly limited, and, for example, a sintered metal filter can be used. In the case of using a sintered metal filter, the filtration diameter of the sintered metal filter is preferably 100 µm or less, more preferably 50 µm or less, further preferably 10 µm or less. When the filtration diameter of the sintered metal filter is 100 µm or less, a fine powder of the catalyst coming out of the first hydrogenation step can be efficiently removed.

Examples of the precipitation include static separation and centrifugation.

### <Second hydrogenation step>

The second hydrogenation step is the step of further hydrogenating, with a fixed-bed reactor or the like, the reaction product in the mixed liquid from which the catalyst and the like have been removed by the solid-liquid separation step. In this step, xylylenediamine can be obtained by hydrogenation of cyanobenzylamine produced as a by-product in the first hydrogenation step, contained in the reaction product, and the purity of xylylenediamine in the reaction product can be enhanced.

The hydrogenation of this step is preferably performed through continuous fixed-bed reaction. For example, a known support metal catalyst, non-supported metal catalyst, Raney catalyst, sponge catalyst, or noble metal catalyst can be used as the catalyst for use in this step. Particularly, a catalyst containing nickel and/or cobalt is preferably used.

When the first hydrogenation step involves a solvent other than liquid ammonia, such as aromatic hydrocarbon, the solvent other than liquid ammonia may be used directly in this step without being separated and removed by the ammonia separation step.

The temperature of the hydrogenation reaction in this step is preferably 30 to 150°C, more preferably 40 to 120°C, further preferably 50 to 100°C. The temperature of 30°C or higher can effectively prevent reduction in conversion rate of cyanobenzylamine. The temperature of 150°C or lower can prevent the progression of nuclear hydrogenation and deamination of xylylenediamine and also effectively prevent the thermal degeneration of the xylylenediamine itself. The hydrogen pressure of the hydrogenation reaction in this step is preferably 0.1 to 10 MPa, more preferably 0.5 to 8 MPa, further preferably 1 to 4 MPa. The hydrogen pressure of 0.1 MPa or higher can effectively prevent reduction in conversion rate of cyanobenzylamine. The hydrogen pressure of 10 MPa or lower can effectively prevent the progression of nuclear hydrogenation and deamination of xylylenediamine.

In the case of performing the second hydrogenation step through continuous reaction, the space velocity (LHSV) of the reaction starting material is preferably 0.1 to 10 h⁻¹, more preferably 0.1 to 3.0 h⁻¹. The space velocity of 0.1 h⁻¹ or more increases an amount that can be treated per time, and is industrially advantageous. The space velocity of 10 h⁻¹ or less can elevate the conversion rate of cyanobenzylamine.

In the case of performing the hydrogenation reaction in this step through batch reaction, the hydrogenation reaction time is preferably 0.5 to 8 hours.

The amount of cyanobenzylamine based on xylylenediamine in a solution obtained after the hydrogenation reaction in this step is preferably kept at 0.02% by mass or less, more preferably at 0.01% by mass or less, further preferably at 0.005% by mass or less, still further preferably at 0.001 % by mass or less. Highly pure xylylenediamine can be obtained by appropriately adjusting reaction conditions such as the temperature, the hydrogen pressure, the reaction time, or the LHSV such that the amount of cyanobenzylamine is kept within the range mentioned above.

### <Purification step>

The production method of the present embodiment may comprise the step of purifying xylylenediamine after the second hydrogenation step. According to the purification step, more highly pure xylylenediamine can be obtained by the purification of the reaction product obtained in the second hydrogenation step.

The purification method in this step is preferably, for example, a method by distillation. This distillation preferably employs a distillation column having a theoretical plate number of 2 or more and more preferably employs a distillation column having a theoretical plate number of 5 or more. The distillation is preferably performed under a reduced pressure condition, and the pressure is preferably 1 to 10 kPa.

### <Cleaning step>

For the production method of the present embodiment, it is preferred to regularly carry out the cleaning step for removing the catalyst attached to the magnet of the magnetic adsorption means in order to prevent the catalyst from causing blockage at subsequent stages, after removing the catalyst powder for a predetermined time by the solid-liquid separation step, as mentioned above.

### <Flow of production method of present embodiment>

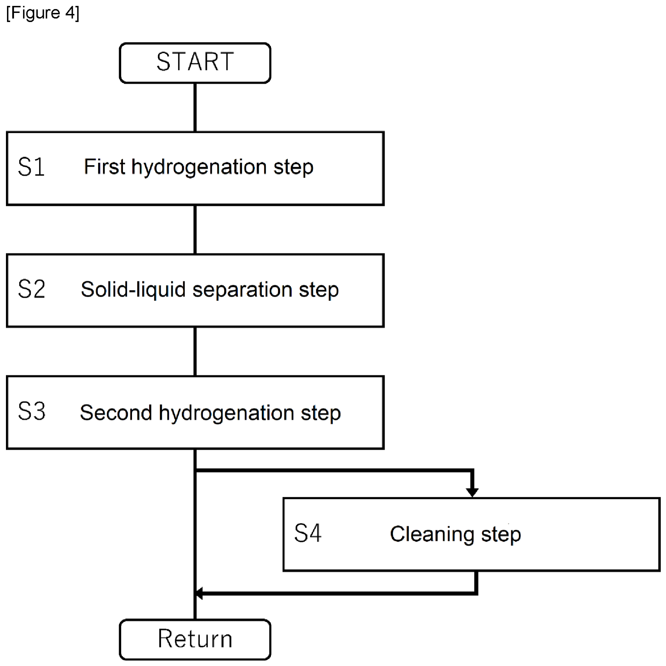

The basic flow of the production method of the present embodiment will be described with reference to Figure 4. Figure 4 is a flow chart showing the basic flow of the present embodiment. As shown in Figure 4, the production method of the present embodiment can comprise the first hydrogenation step and the solid-liquid separation step as well as the second hydrogenation step and the cleaning step. In Figure 4, a form using one magnet strainer is illustrated as an example.

When the production method of the present embodiment is started as shown in Figure 4, as shown in step S1, dicyanobenzene is first hydrogenated in the presence of a catalyst to obtain a mixed liquid containing at least a reaction product and at least a portion of the catalyst, in the first hydrogenation step. The first hydrogenation step can be carried out with, for example, a hydrogenation reactor. The mixed liquid obtained in the first hydrogenation step is sent to the solid-liquid separation step, so that step S2 is performed.

As shown in the step S2, the mixed liquid obtained in the first hydrogenation step is sent to the solid-liquid separation step. In the solid-liquid separation step, the mixed liquid is supplied into the magnet strainer to separate the catalyst in the mixed liquid by the magnetic adsorption means. The mixed liquid from which solid matter such as the catalyst has been separated in the solid-liquid separation step is sent to the second hydrogenation step, so that step S3 is performed. Although not shown in a drawing, the ammonia separation step can be carried out as a step previous (between the step S1 and the step S2) or a step subsequent (between the step S2 and the step S3) to the solid-liquid separation step, as mentioned above, to remove liquid ammonia in the mixed liquid.

As shown in the step S3, the mixed liquid from which solid matter such as the catalyst has been removed in the solid-liquid separation step is sent to the second hydrogenation step. In the second hydrogenation step, the reaction product in the mixed liquid is further hydrogenated with a fixed-bed reactor or the like. In the second hydrogenation step, the purity of xylylenediamine in the reaction product can be enhanced by hydrogenation of cyanobenzylamine produced as a by-product in the first hydrogenation step, contained in the reaction product. Xylylenediamine produced by the step S3 is subjected, if necessary, to the purification step or the like and then shipped as a product.

In the production method of the present embodiment, the step S1 to the step S3 are performed continuously and repetitively, and after a lapse of a predetermined period, step S4 (cleaning step) is performed. In the step S4, as mentioned above, the cleaning method (cleaning step) of the present embodiment is carried out in order to remove the catalyst and the like attached to the surface of the magnetic adsorption means in the magnet strainer. In the production method of the present embodiment, the cycle at which the cleaning step is carried out is not particularly limited and is appropriately determined depending on the scale of an apparatus or the amount of xylylenediamine produced. In the production method of the present embodiment, the step S1 is performed again after the completion of the cleaning step, so that the xylylenediamine production step is started. In Figure 4, an example using one magnet strainer is illustrated. However, as mentioned above, the production method of the present embodiment can clean the magnet strainer without the cessation of the xylylenediamine production step, by using a plurality of magnet strainers and closing respective valves at the inlet and the outlet of the magnet strainer to be cleaned while holding the other magnet strainer(s) in action. In this case, the steps S1 to S3 and the step S4 are carried out in parallel.

Although the production method and the cleaning method of the present embodiment are described above, the present invention is not limited by the description mentioned above.

### Examples

Hereinafter, the production method of the present embodiment will be specifically described. However, the present invention is not limited by the following Examples.

### [Example 1]

The first hydrogenation step and the solid-liquid separation step were carried out using a magnet strainer having the structure shown in Figure 1 to synthesize meta-xylylenediamine from a mixed liquid of isophthalonitrile and liquid ammonia as a starting material. After liquid passing of 610 kg in total of the reaction liquid, upstream and downstream valves of the strainer were closed to stop the flow of the mixed liquid. Then, a lower drain valve of the strainer was opened, and a residual liquid was drained off.

Then, a bolt of an upper lid portion was removed, and the lid was displaced. A part provided with magnet rods (magnetic adsorption means) was taken out. Then, the magnetic adsorption means was washed by washing off, with water, a fine powder attached to the magnet rods, and wiping the magnet rods with waste cloth. The total operating time required for the cleaning of the magnet strainer was 30 minutes, and two people were required for the operation.

### [Comparative Example 1]

The first hydrogenation step and the solid-liquid separation step were carried out using a conventional magnet strainer having a structure having a magnet portion connected to a lid portion to synthesize meta-xylylenediamine from a mixed liquid of isophthalonitrile and liquid ammonia as a starting material. After liquid passing of 610 kg in total of the reaction liquid, upstream and downstream valves of the strainer were closed to stop the flow of the mixed liquid. Then, a lower drain valve of the strainer was opened, and a residual liquid was drained off.

Then, a bolt of the upper lid portion was removed, and a chain block was mounted to a handle. The lid was dragged upward by 70 to 80 cm until the magnet portion (magnetic adsorption means) was exposed. The lid was pushed aside while held, and the magnetic adsorption means was washed by washing off, with water, a fine powder attached to the magnet portion, and then wiping the magnet portion with waste cloth. The total operating time required for the cleaning of the magnet strainer was 1 hour, and three people were required for the operation. Thus, more time and more manpower were required as compared with Example 1.

The disclosure of Japanese Patent Application No. 2023-058898 filed on March 31, 2023 is incorporated herein by reference in its entirety.

All literatures, patent applications, and technical standards described herein are incorporated herein by reference to the same extent as that when it is specifically and individually described that the individual literatures, the patent applications, and the technical standards are incorporated herein by reference.

### Reference Signs List

100: magnet strainer, 10: main body, 12: opening, 20: lid material, 30: magnetic adsorption means, 32: holding member, 34: magnet, 36: magnet member, 40: guide, 50: bucket

## Claims

1. A xylylenediamine production method by hydrogenation of dicyanobenzene, the xylylenediamine production method comprising the steps of:
hydrogenating the dicyanobenzene in the presence of a catalyst to obtain a mixed liquid containing at least a reaction product and at least a portion of the catalyst; and
passing the mixed liquid through a magnet strainer to separate the catalyst by magnetic adsorption, wherein
the magnet strainer comprises: a main body having an opening; a lid material placed on the opening of the main body; and a magnetic adsorption means disposed inside the main body and separated from the lid material.

2. The xylylenediamine production method according to claim 1, wherein the magnet strainer comprises, inside the main body, a guide that supports the magnetic adsorption means, and a bucket for removing solid matter in the mixed liquid.

3. The xylylenediamine production method according to claim 1, wherein the magnetic adsorption means comprises a magnet and a holding member that holds the magnet.

4. The xylylenediamine production method according to claim 1, wherein
the magnet strainer comprises, inside the main body, a guide that supports the magnetic adsorption means, and a bucket for removing solid matter in the mixed liquid,
the magnetic adsorption means comprises a magnet and a holding member that holds the magnet, and
the holding member of the magnetic adsorption means is supported by the guide.

5. The xylylenediamine production method according to any one of claims 1 to 4, further comprising the step of taking out the magnetic adsorption means separated from the lid material of the main body of the magnet strainer, followed by the cleaning of the magnetic adsorption means.

6. A magnet strainer cleaning method for removing a catalyst by magnetic adsorption from a mixed liquid containing a reaction product and the catalyst, the mixed liquid being obtained by hydrogenation of dicyanobenzene in the presence of the catalyst, wherein
the magnet strainer comprises: a main body having an opening; a lid material placed on the opening of the main body; and a magnetic adsorption means disposed inside the main body and separated from the lid material, and
the magnetic adsorption means separated from the lid material is taken out of the main body of the magnet strainer, followed by the cleaning of the magnetic adsorption means.
